(19) Europäisches Patentamt European Patent Office Office européen des brevets

(11) **EP 4 033 268 B1**

(12) **EUROPEAN PATENT SPECIFICATION**

(45) Date of publication and mention of the grant of the patent:
**22.11.2023 Bulletin 2023/47**

(21) Application number: **21305089.1**

(22) Date of filing: **25.01.2021**

(51) International Patent Classification (IPC):
*G01S 11/02* (2010.01)  *G01S 11/06* (2006.01)
*G01S 5/00* (2006.01)  *G16H 50/80* (2018.01)
*H04W 24/08* (2009.01)  *H04L 69/28* (2022.01)
*G01S 5/02* (2010.01)  *H04W 4/02* (2018.01)

(52) Cooperative Patent Classification (CPC):
**G01S 11/06; G01S 5/01; G16H 50/80; H04L 69/28; H04W 4/023; H04W 24/08;** G01S 5/0284

(54) **METHOD, COMPUTER PROGRAM AND SYSTEM FOR CONTAMINATION LIKELIHOOD ASSESSMENT FOR A PERSON OF INTEREST**

VERFAHREN, COMPUTERPROGRAMM UND SYSTEM ZUR KONTAMINATIONSWAHRSCHEINLICHKEITSBEURTEILUNG FÜR EINE INTERESSIERENDE PERSON

PROCÉDÉ, PROGRAMME INFORMATIQUE ET SYSTÈME D'ÉVALUATION DE LA PROBABILITÉ DE LA CONTAMINATION D'UNE PERSONNE D'INTÉRÊT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**27.07.2022 Bulletin 2022/30**

(73) Proprietor: **Bull SAS**
**78340 Les Clayes-sous-Bois (FR)**

(72) Inventors:
• **BENRACHI, Samia**
**13127 VITROLLES (FR)**
• **WAWRZYNIAK, Piotr**
**88-200 Radziejów (PL)**
• **MATUSIAK, Robert**
**87-100 Torun (PL)**
• **FIEREMANS, Geert**
**Boca Raton, FL Florida 33496 (US)**

(74) Representative: **IPAZ**
**Bâtiment Platon**
**Parc Les Algorithmes**
**91190 Saint-Aubin (FR)**

(56) References cited:
• **Cencetti G. ET AL: "Digital Proximity Tracing in the COVID-19 Pandemic on Empirical Contact Networks", medRxiv, 2 July 2020 (2020-07-02), pages 1-49, XP055796873, DOI: 10.1101/2020.05.29.20115915 Retrieved from the Internet: URL:https://www.medrxiv.org/content/10.1101/2020.05.29.20115915v2.full.pdf [retrieved on 2021-04-19]**
• **TANG XIAOYONG ET AL: "Indoor Crowd Density Estimation Through Mobile Smartphone Wi-Fi Probes", IEEE TRANSACTIONS ON SYSTEMS, MAN, AND CYBERNETICS: SYSTEMS, IEEE, PISCATAWAY, NJ, USA, vol. 50, no. 7, 1 July 2020 (2020-07-01), pages 2638-2649, XP011793663, ISSN: 2168-2216, DOI: 10.1109/TSMC.2018.2824903 [retrieved on 2020-06-16]**
• **DAMIN ZHANG ET AL: "SIRS Epidemic Model with Immunization on Sparsely Distributed Regular Lattice", NETWORKING AND DIGITAL SOCIETY, 2009. ICNDS '09. INTERNATIONAL CONFERENCE ON, IEEE, PISCATAWAY, NJ, USA, 30 May 2009 (2009-05-30), pages 133-136, XP031478507, ISBN: 978-0-7695-3635-4**

**Description**

**Field of the invention**

The present invention relates to a method for contamination likelihood assessment. It also relates to a computer program and to a system configured to implement such a method.

The field of the invention is the field of the assessment of contamination likehood for diseases, such as viral contamination, between individuals, in particular in the event of a pandemic.

**Background**

In a self-confinement environment, for the purpose of limiting the contamination, it is required to trace the suspected cases, in order to provide specific assistance as the case could degenerate and require immediate assistance rapidly and become a severe case.

The most common and convenient technique to trace the movement of the suspected case is based on the use of a specific tracing application installed in a user device such as a Smartphone. The Smartphone with the specific tracing application emits a signal with a unique ID and is configured to receive the signals emitted by other user devices. The Received Signal Strength Indication (RSSI) for a detected signal is used to determine distance between two individuals, and assess whether there might be a contamination when one of the two individuals is carrier of a virus.

But, the inventors of the present invention have noticed that the distance evaluation based only on the RSSI is not accurate enough to distinguish people in near proximity, and especially below 2 meters. For example, a purely RSSI-based distance calculation for Bluetooth is only accurate somewhere between half and double of the actual distance. Hence, using received RSSI values is not enough to decide whether a Bluetooth emitting device is less than 2 meters away from a receiving user device. The RSSI received for an emitting device that is exactly 2 meters away, will after calculation lead to a received distance somewhere between 1 and 4 meters (or 6 dB in terms of RSSI value).

Therefore, the prior art techniques for contamination likelihood assessment are not accurate enough et provide approximate results.

A purpose of the present invention is to overcome at least one of these drawbacks.

Another purpose of the invention is to propose a contamination likelihood assessment technique that provides more accurate results.

Yet another purpose of the present invention is to propose a contamination likelihood assessment technique that is more efficient to detect and/or trace suspected cases.

**Summary of the invention**

The present invention makes it possible to achieve at least one of these aims by a method, according to independent claim 1, for the evaluation of a contamination likelihood for a person of interest (POI), carrying a user device configured to detect signals with personal IDs emitted by other user devices, said method comprising the following steps:

- determining a duration, called exposure duration, comprising at least one time window(s), of a predetermined length, during which the user device of said POI detected at least one signal, called contaminated person's signal, emitted from the user device of at least one contaminated person, and
- for each time window, assessing a contamination likelihood for said POI according to:

   ▪ a received signal strength indication (RSSI) value, for the contaminated person's signal, relative to the distance between said contaminated person and the POI, and
   ▪ an RSSI threshold value, representing a contamination distance below which contamination is possible;

wherein said method comprises using at least one crowd attribute characterizing the crowd around said POI during at least one of said time window(s), to adjust the received RSSI value and/or the RSSI threshold value.

Thus, like the prior art techniques, the present invention provides a method where the contamination likelihood of a POI is based on the received RSSI value of the signal emitted by the user device of at least one contaminated person, representative of the distance between the POI and the contaminated person, and a RSSI threshold value, representative of a contamination distance below which contamination is possible. If the received RSSI value corresponds to a distance that is below, or equal to, the distance corresponding to the threshold value, then it is likely that the POI may be contaminated. In the contrary, it is likely that the POI may not be contaminated.

But, unlike the prior art techniques, the present invention uses at least one attribute of the crowd around the POI, during at least one time window, in order to adjust the value of the RSSI threshold or the value of the received RSSI. In other words, the invention seeks to take into consideration the environment of the POI, and more particularly the composition of the crowd around the POI, in order to assess whether the distance between the POI and the contaminated person is below, or equal to, the threshold distance. Thus, the present invention makes it possible to use a more accurate value representative of the contamination distance, and/or a more accurate value representative of the distance between the POI and the contaminated person. Indeed, for example a same received RSSI value doesn't correspond to the same distance in a crowded environment or in a not crowded environment. As a consequence, the present invention provides more accurate results in detecting contamination situations and is more efficient to trace suspected cases.

According to the invention, at least two, in particular all the, time windows may have the same duration.

Alternatively, or in addition, at least two, in particular all the, time windows may have different durations.

According to a non-limitative example, at least one time window may last one minute.

According to embodiments, the at least one crowd attribute may be used to adjust the received RSSI value. In this case, the assessment of the contamination likelihood is carried out by taking into account the adjusted received RSSI value.

In these embodiments, the method according to the invention may comprise, for each time window, a step for adjusting the received RSSI value for the contaminated person's signal.

The adjusted received RSSI value may be greater or smaller than the received RSSI value. In most cases though, the adjusted received RSSI value is greater than the received RSSI value, since the RSSI values are generally negative values.

Alternatively, or in addition, the at least one crowd attribute may be used to adjust the RSSI threshold value.

In this case, the method according to the invention may comprise a step calculating an adjusted RSSI threshold value as function of:

- the at least one attribute of the crowd, and
- a given RSSI cutoff value, representing a contamination distance for the user device of the POI.

The RSSI cutoff value is generally specific to the user device, or the model of user device. The RSSI cutoff value may given for a particular model of user device, especially by a provider or a manufacturer of the user device.

The RSSI cutoff value corresponds to a given distance. Generally, the given RSSI cutoff value corresponds to a distance of 2 meters.

According to an advantageous feature of the present invention, a first crowd attribute, called crowd diversity, may be used to adjust the received RSSI value, and/or the RSSI threshold value.

The crowd diversity relates to change of persons composing the crowd around said POI during at least one time window.

In other words, the crowd diversity may indicate if the crowd around the POI is:

- static, i.e. the persons composing the crowd are not changing, like for example in the office or in a classroom where people are static, or
- changing, i.e. the persons composing the crowd are changing dynamically, like for example in a train station where people are moving and different people are passing by)

In particular, the crowd diversity may be used to adjust the RSSI threshold value to a lower value, or the received RSSI value to a higher value

Indeed, the inventors of the present invention have noticed that the diversity of the crowd around a person may modify the contamination risk for this person, from a contaminated person. For example, the risk of contamination of a POI by a contaminated person is lower when people around the POI are moving than when people around the POI are static.

On contrary, risk of virus transmission from contaminated person is larger when the crowd is static, i.e. this person is not moving and is still close, for example in the same room, than when the crowd is dynamic, i.e. sick person is encountered for only short period of time. This assumes crowd density to be constant of course.

The method according to the invention may comprise a step for determining the crowd diversity for at least one, especially for each, time window.

In a non-limitative exemplary embodiment, the step for determining the crowd diversity may comprise the following operations:

- determining a first list of unique ID(s) received by the user device of the POI, during said time window,
- determining a second list of unique ID(s) received by the user device of the POI, during the time window immediately preceding said time window, and

- calculating said crowd diversity for said time window as, or as a function of, the symmetric difference between said first and second lists of unique ID(s).

Such a step for determining the crowd diversity is easy to implement because it only uses the signals received by the user device of the POI and doesn't need additional measurements, nor additional sensors. Obviously, it has to be noted that, with such an exemplary step, the method according to the invention can't determine crowd diversity for the first time window in the exposure duration. But, this is not very detrimental because the environment around the POI generally doesn't change a lot between two consecutive time windows.

In non-limitative embodiments of the method according to the invention, for at least one, and in particular for each, time window, the crowd diversity value determined for said time window may be used to adjust the received RSSI value and/or the RSSI threshold value.

In alternative embodiments of the method according to the invention, for at least one, and in particular for each, time window, an average value, called mean crowd diversity, of the crowd diversities determined for several time windows, and in particular for all the time windows, of the exposure duration, may be used to adjust the received RSSI value, and/or the RSSI threshold value.

In this case, the method according to the invention may first determine the mean crowd diversity value before the adjustment of the received RSSI value and/or the RSSI threshold value.

According to an example, the mean crowd diversity may be the mathematical mean of the crowd diversities for several, and in particular all, time windows of the exposure duration.

According to another example, the mean crowd diversity may correspond to another value calculated with a predetermined formula that is different from the mathematical mean. Such a formula may take into account different parameters such as the length of the exposure duration, or the length of each time window.

According to an advantageous feature, a second crowd attribute, called crowd density, may be used to adjust the received RSSI value, and/or the RSSI threshold value.

The crowd density relates to the density of persons in the crowd around said POI during at least one time window.

In particular, the crowd density may be used to adjust the RSSI threshold value to a higher value, or the received RSSI value to a lower value.

Indeed, the inventors of the present invention have noticed that the density of the crowd around a POI may modify the contamination risk for this POI, from a contaminated person. For example, the risk of contamination of a POI by a contaminated person is greater in a situation where the density of people is higher compared to a situation where the density of people is lower. Indeed, in a very dense situation, people are very close to each other so that they contaminate each other more rapidly and easily.

In this case, the method according to the invention may comprise a step for determining the crowd density for at least one, and especially for each, time window.

In a non-limitative exemplary embodiment, such a step for determining the crowd density for a time window may comprise the following operations:

- determining a total number of unique ID(s) received by the user device of the POI, during said time window,
- calculating a density value by dividing said total number of unique ID(s) by a reception range value, representing the reception range of the POI's user device, and
- determining a crowd density value for said time window, as a function of said density value.

The reception range value is generally associated with the user device model and is provided by the manufacturer of the user device.

Such a step for determining the crowd diversity is easy to implement because it only uses the signals received by the user device and doesn't need additional measurements, nor additional sensors.

According to some embodiments, the crowd density may be equal to the calculated density value.

According to non-limitative embodiments, the crowd density may be a value, determined beforehand, and associated to a different class of density. Each density class may be defined by a lower and a higher threshold values of density. In this case, the density value is used to determine a class crowd density class and the crowd density is given by the value defined for this class.

In a non-limitative example, the following crowd density classes may be defined, with, for each class, associated calculated density values and a crowd density value:

| Crowd density value | Crowd density class | Calculated density value V |
|---|---|---|
| 1 | Nearly Empty | $V<0,05$ person/m$^2$ |

(continued)

| Crowd density value | Crowd density class | Calculated density value V |
|---|---|---|
| 2 | Very low | $0,05 < V < 0,2$ person/m$^2$ |
| 4 | low | $0,2 < V < 0,3$ person/m$^2$ |
| 6 | moderate | $0,3 < V < 0,4$ person/m$^2$ |
| 8 | high | $0,4 < V < 1,10$ person/m$^2$ |
| 12 | Very high | $1,0 < V < 2,0$ person/m$^2$ |
| 20 | Extremely high | $0,2 < V$ person/m$^2$ |

Of course, these values and classes are given for the sake of example only and are in no way limitative.

In non-limitative embodiments of the method according to the invention, for at least one, and in particular for each, time window, the crowd density value determined for said time window may be used to adjust the received RSSI value, and/or the RSSI threshold value.

In alternative embodiments of the method according to the invention, for at least one, and in particular for each, time window, an average value, called mean crowd density, of the crowd densities for several time windows, and in particular for all time windows, of the exposure duration, may be used to adjust the received RSSI value, and/or the RSSI threshold value.

Thus the same value of the crowd density may be used for several, and especially all, time windows in the exposure duration.

In this case, the method according to the invention may first determine the mean crowd density value before the adjustment of the received RSSI value and/or the RSSI threshold value.

According to an example, the mean crowd density may be calculated as the mathematical mean of the crowd densities for several, and in particular for all, time windows.

According to another example, the mean crowd density may be the most frequent crowd density value for all time windows of the exposure duration. In an example based to the classes defined above, if the most frequent crowd density value is 20, i.e. "extremely high density", then the mean crowd density is equal to 20, even if there are some time windows for which the crowd density is not equal to 20.

When the method according to the invention uses an adjusted RSSI threshold value, the adjusted RSSI threshold value may be determined by the following formula:

$$\text{Adjusted RSSI threshold} = \text{base RSSI cutoff} + \frac{base\ RSSI\ cutoff}{mean\ crowd\ civersity^2} * mean\ crowd\ density$$

with:

- base RSSI cutoff : the RSSI cutoff value for a contamination distance for the POI's user device: this value is generally provided by the manufacturer of the user device, or deducted from the specification of the user device;
- *mean crowd diversity* : the average crowd diversity value; and
- *mean crowd density* : the average crowd density value.

In this case, the adjustment uses average values for crowd density and crowd diversity. Thus the adjusted RSSI value is common to several, especially to all, time windows of the exposure duration.

In alternative embodiments, when the method according to the invention uses an adjusted RSSI threshold value, the adjusted RSSI threshold value may be determined by the following formula:

$$\text{Adjusted RSSI threshold}_i = \text{base RSSI cutoff} + \frac{base\ RSSI\ cutoff}{crowd\ diversity_i^2} * crowd\ density_i$$

with:

- base RSSI cutoff: a given RSSI Cutoff value for a contamination distance for the POI's user device: this value is generally provided by the manufacturer of the user device, or deducted from the specification of the user device;

- $crowd\ diversity_i^2$ : the crowd diversity value for time window "i", and
- $crowd\ density_i$: the crowd density value for time window "i".

In this case, the adjusted RSSI threshold value is individual for at least one, and in particular for each, time window.

In a preferred embodiment, the signal emitted by each user device is a Bluetooth ® signal.

Of course, the signal emitted by each user device may also not be a Bluetooth ® signal. The signal emitted by each user device may generally be of any type of wireless signal, such as radiofrequency signal, a Wi-Fi signal, a Li-Fi signal, etc.

According to non-limitative embodiments, if the exposure duration comprises at least one time window for which:

- the received RSSI value, or the adjusted received RSSI value when applicable, is lower than or equal to the RSSI threshold value, or the adjusted RSSI threshold value when applicable, the risk of contamination is high and the POI is considered as a suspected case; and
- in the contrary, it is considered that the risk of contamination is low and the POI is not considered as a suspected case.

According to another aspect of the same invention, it is proposed a computer program, as defined in independent claim 10, comprising instructions which, when executed by at least one data processing device, cause said at least one data processing device to carry out the method according to the invention for the evaluation of contamination likelihood for a person of interest (POI).

The computer program may be coded by using any kind of coding language such as C, C++, Java, Python, etc.

The computer program may be stored in a data processing device or machine, in the coding language or in machine language.

The computer program may be stored in storage medium that can be read by a data processing device or machine such as memory stick, USB dongle, a hard drive, etc.

The computer program may comprise a first computer program, called client application, provided to be installed in, and executed by, the user device of the POI.

The computer program may comprise a second computer program, called server application, provided to be installed in, and executed by, a central server remote from the user device of the POI.

According to another aspect of the present invention it is proposed a method, as defined in independent claim 11, for detecting persons suspected of contamination, in case of a contamination between persons, in particular in the event of a pandemic, said method comprising:

- equipping persons with user devices, each configured to emit a signal with a personal ID and detect signals with personal ID emitted by other user devices;
- evaluating the contamination likelihood of at least one of said persons by the method according to the present invention for the evaluation of a contamination likelihood for a person of interest (POI).

For at least one, in particular each, person, the assessment of the contamination likelihood may be carried totally by the user device of the person, especially by a computer program installed in said user device.

For at least one, in particular each, person, the assessment of the contamination likelihood may be carried:

- partly by the user device, especially by a computer program, called client application, installed in said user device; and
- partly by central server, especially by a computer program, called server application, installed in said server, in communication with said user device through a communication network such as the Internet.

According to another aspect of the present invention, it is proposed a system, as defined in independent claim 12, for the evaluation of a contamination likelihood for a person of interest (POI), said system comprising:

- a user device, provided for being carried out by the POI and configured to detect signals with personal ID(s) emitted by other user devices, and
- a computer program, called client application, installed in said user device;

configured to carry out the steps of the method according to the present invention for the evaluation of a contamination likelihood for said person of interest (POI).

According to a first embodiment, all the steps of the method may be carried out in said user device. In this case, the system for the evaluation of a contamination risk, is constituted by the user device equipped with the client application.

According to another embodiment, the system for the evaluation of a contamination risk according to present invention further comprises

- a central server, in communication with the user device, and
- a computer program, called server application, installed in said central;

said server application being configured to carry out, together with the user device and the client application installed therein, the steps of the method according to the present invention for the evaluation of a contamination risk for said person of interest (POI).

According to another aspect of the present invention, and as defined in independent claim 14, it is proposed a system for detecting persons suspected of contamination, in case of a contamination between persons, in particular in the event of a pandemic, said system comprising:

- for each of said persons:

    ▪ a user device configured to emit a signal with a personal ID and detect signals with personal ID emitted by other user devices, and
    ▪ a computer program, called client application, installed in said user device; and

- for several, in particular for all, persons:

    ▪ a central server, in communication with the user device, and
    ▪ a computer program, called server application, installed in said central server;

configured to carry out the steps of the method according to the present invention for the evaluation of contamination likelihood for at least one of said persons.

In the present invention, each user device may be a Smartphone, a tablet, a personal computer, etc.

Each user device may communicate with the central server through the Internet.

## Description of the figures and embodiments

Other advantages and characteristics will become apparent on examination of the detailed description of an embodiment which is in no way limitative, and the attached figures, where:

- FIGURE 1 is a diagrammatic representation of a non-limitative example of an example of an exposure duration in the sense of the present invention;
- FIGURE 2 is a schematic representation of a non-limitative example of a method according to the invention for contamination likelihood assessment for a person of interest (POI);
- FIGURE 3 is a schematic representation of a non-limitative example of a step that may be used in the present invention for determining crowd diversity for a given time window;
- FIGURE 4 is a schematic representation of a non-limitative example of a step for determining crowd density for a given time window, that may be used in the present invention;
- FIGURE 5 is a schematic representation of a non-limitative example of a system according to the invention for contamination likelihood assessment for a person of interest (POI);
- FIGURE 6 is a schematic representation of another non-limitative example of a system according to the invention for contamination likelihood assessment for a person of interest (POI); and
- FIGURE 7 is a schematic representation of a non-limitative example of a system according to the invention for detecting persons suspected of contamination, in case of a contamination between persons, in particular in the event of a pandemic.

It is well understood that the embodiments that will be described below are in no way limitative. In particular, it is possible to imagine variants of the invention comprising only a selection of the characteristics described hereinafter, in isolation from the other characteristics described, if this selection of characteristics is sufficient to confer a technical advantage or to differentiate the invention with respect to the state of the prior art. Such a selection comprises at least one, preferably functional, characteristic without structural details, or with only a part of the structural details if this part alone is sufficient to confer a technical advantage or to differentiate the invention with respect to the prior art.

In the figures, elements common to several figures retain the same reference.

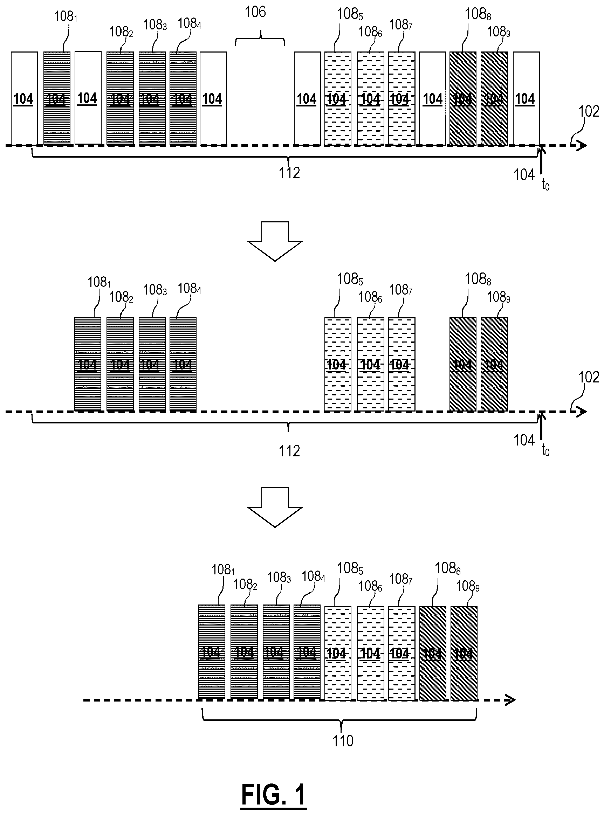

FIGURE 1 is a schematic representation of a non-limitative example of an exposure duration in the sense of the present invention.

In the FIGURE 1, the arrow 102 represents the timeline, and $t_0$ corresponds to the current time on the timeline.

As illustrated on FIGURE 1, the timeline 102 is divided in several time windows 104, in the past compared to current time, during which the user device of a person of interest (POI) is activated to detect a signal with personal ID emitted by other user devices, each of which is carried out another person that is in close proximity of the POI.

For each time window, and for each received signal, the user device of the POI, or the application installed on said user device, may be configured to store:

- the Received Signal Strength Indication (RSSI) value for said received signal, and
- the ID contained in said received signal.

According to a non-limitative example, each time window lasts 1 minute and two consecutive time windows are separated by a period of 10 seconds.

The time windows 104 are not necessary contiguous in time. For example, as illustrated in FIGURE 1, there is no time window during the period 106.

The time windows 104 shown in FIGURE 1 have the same duration. But, according to the invention, the time windows may also have different durations.

During several time windows 104, noted $108_1$-$108_9$, the user device of the POI has detected the ID associated to at least one sick person. For example:

- during four time windows $108_1$-$108_4$, the user device of the POI has detected the ID associated to at least one sick person. It is to be noted that one of the time windows $108_1$-$108_4$, i.e. time window $108_1$ is not contiguous with the other three time windows $108_2$-$108_4$;
- during three time windows $108_5$-$108_7$, the user device of the POI has detected the ID associated to at least one sick person;
- during two time windows $108_8$-$108_9$, the user device of the POI has detected the ID associated to at least one sick person;

At least one sick person encountered during the time windows 108 may be the same person or different persons.

During at least one of the time windows 108, the POI may encounter one or several sick persons. When, during a time window, several sick persons are encountered the contamination likelihood for said time window is assessed for each sick person individually.

According to a non-limitative example, all the time windows 104 considered to determine the exposure direction are the time windows during a predetermined period of time, referenced 112. This predetermined period of time may be, for example, the incubation period of a virus in case of contamination by a virus.

The exposure duration 110 is obtained by taking into account all the time windows 108 during which the ID associated to at least one contaminated person is detected by the user device of POI.

In the example shown in FIGURE 1, the exposure duration 110 corresponds to the duration obtained by putting together all of the time windows 108. Thus, the exposure duration 110 comprises nine time windows $108_1$-$108_9$.

The method according to the invention takes into account these time windows 108. It is recalled that, for each time window 108, and for each signal received during each time window 108, the following information are stored:

- the Received Signal Strength Indication (RSSI) value for said received signal, and
- the personal ID contained in said received signal.

These information is used in the method according to the invention in order to determine the risk of contamination for the POI.

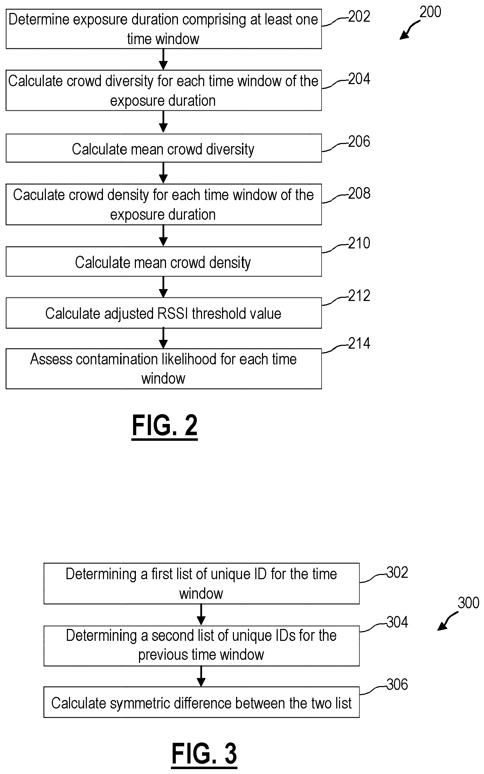

FIGURE 2 is a schematic representation of a non-limitative example of a method according to the invention for contamination likelihood assessment for a person of interest (POI).

The method 200, represented in FIGURE 2, is executed for a person of interest (POI) in order to determine whether the POI might have been contaminated by an encountered contaminated/sick person. In order to implement the method 200, the POI carries a user device configured to detect the signals emitted by the user devices carried by other persons encountered by the POI. The signal emitted by the user device of each person contains an identification data, called personal ID, that is associated and individual to this person.

According to the invention, the personal ID may be an ID of the user device, or an ID of the person holding the user device, or the ID of a person for an application installed in the user device.

The personal ID may be an anonymized ID so that the identity of the user can't be read through the data composing ID.

The method 200 comprises a first step 202 during which an exposure duration is determined. The exposure duration corresponds to one or several time windows during which the user device of the POI detected the ID associated to a contaminated person. For example, the exposure duration may be determined as described above with respect to FIGURE 1. Of course, the example given in FIGURE 1 is in no way limitative and the exposure duration may be determined in a different way. Step 202 provides an exposure duration comprising one or several time windows, such as the exposure duration 110 of FIGURE.

After step 202, the method 200 comprises steps for determining at least one crowd attribute. The at least one crowd attribute will be used in the assessment of the contamination likelihood, by adjusting:

- the received RSSI value for the signal emitted by the user device of a contaminated person: this value is representative of the distance between the contaminated person and the POI; or
- a RSSi threshold value: this value is representative of threshold distance, below which it is considered that the POI is contaminated by sick person.

A first crowd attribute may be the crowd diversity, representing the diversity of the crowd during a time window, i.e. if the crowd around the POI is changing or not, i.e. the composition of the crowd is static or dynamic.

The method comprises a step 204 determining crowd diversity for each time window (except for the first time window $108_1$ with the example of FIGURE 3, i.e. time window $108_1$ in the example of FIGURE 1). An example of crowd diversity calculation is given below with respect to FIGURE 3. It is important to note that, for a time window, the crowd diversity is calculated by taking into account all the received signals during said time window, and not only the contaminated person(s) signal(s).

A step 206 calculates a mean crowd diversity as the arithmetic mean of the crowd diversities obtained for all the time windows (except for the first time window $108_1$ with the example of FIGURE 3).

A second crowd attribute may be the crowd density, representing the crowd density for a time window.

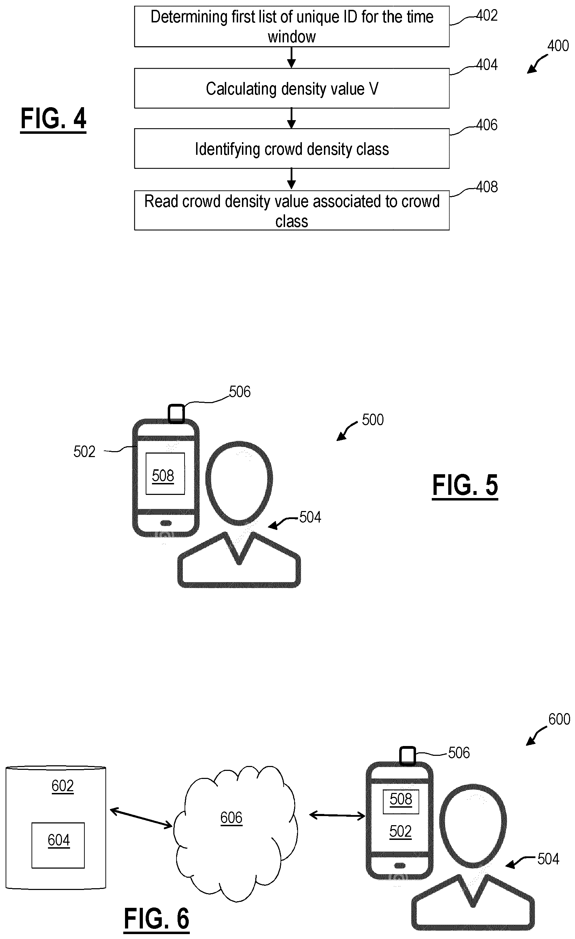

The method comprises a step 208 determining crowd density value for each time window. A non-limitative example of crowd diversity calculation is given below with respect to FIGURE 4. It is important to note that, for a time window, the crowd density is calculated by taking into account all the received signals during said time window, and not only the signal(s) received from the user device of contaminated persons.

Then a step 210 calculates a mean crowd density as the most frequent crowd density encountered for all the time windows of the exposure duration.

During step 212, an adjusted RSSI threshold value is calculated as a function of the mean crowd diversity and the mean crowd density. For example, the adjusted RSSI threshold may be calculated using the following formula:

$$\text{Adjsuted RSSI threshold} = \text{base RSSI cutoff} + \frac{base\ RSSI\ cutoff}{mean\ crowd\ civersity^2} * mean\ crowd\ density$$

with:

- base RSSI cutoff : the RSSI cutoff value for a contamination distance for the POI's user device: this value is generally provided by the manufacturer of the user device;
- Adjusted RSSI threshold : the adjusted RSSI threshold value;
- *mean crowd diversity* : the average crowd diversity value; and
- *mean crowd density* : the average crowd density value.

In this case, the adjustment uses average values for crowd density and crowd diversity. Thus the adjusted RSSI value is common to all time windows.

According to an alternative embodiment, the adjusted RSSI threshold may be calculated individually for each time window as a function of the crowd diversity and the crowd density for said time window. In this alternative embodiment, steps 206 and 210 calculating mean values for crowd diversity and crowd density might not be carried out.

After step 212, a step 214 assesses the contamination likelihood for each time window. During this step, the received RSSI value for the signal received from the user device of the contaminated/sick person is compared to the adjusted RSSI threshold value obtained at step 212:

- if the received RSSI value is lower than or equal to the adjusted RSSI threshold value then the risk of contamination is high and the POI is considered as being likely contaminated; and
- in the contrary, it is considered that the risk of contamination is low and the POI is not considered as likely contaminated.

In the example described with reference to FIGURE 2, the crowd attributes are used to adjust the RSSI threshold value. According to an alternative embodiment, at least one crowd attribute may be used to adjust the received RSSI value for the signal received from the user device of the contaminated person. In this alternative embodiment, step 212 carries out such an adjustment using a predetermined formula.

In the example described with reference to FIGURE 2, the used crowd attributes are crowd diversity and crowd density. According to an alternative embodiment, only one of crowd diversity and crowd density may be used. According to another embodiment, a different crowd attribute may be used, in addition or in place of the crowd attribute used in this example.

FIGURE 3 is a schematic representation of a non-limitative example of a step for determining crowd diversity for a given time window, that may be used in the present invention.

The step 300, represented in FIGURE 3, may be the step 204 in the method 200 of FIGURE 2.

The step 300 comprises a first step 302 determining a first list of unique ID(s) received by the user device of the POI, during the given time window for which the crowd diversity is calculated. This first step gives a list of unique IDs.

The step 300 comprises a second step 304 determining a second list of unique ID(s) received by the user device of the POI, during the time window immediately preceding said given time window, i.e. the time window just before the given time window for which the crowd diversity is calculated.

For example, if the time window for which the crowd diversity is calculated is the time window $108_5$, then the preceding time window is time window $108_4$. In this example, step 302 provides a first list of unique IDs obtained for the time window $108_5$ and step 304 provides a second list of unique IDs obtained for time window $108_4$.

After step 304, a step 306 calculates the crowd diversity for the given time window as the symmetric difference between said first and second lists of unique IDs.

Step 300, described here with reference to FIGURE 3, is only a non-limitative advantageous example for calculating the crowd diversity for a time window. Of course, other techniques for calculating crowd diversity may be used in the present invention.

FIGURE 4 is a schematic representation of a non-limitative example of a step for determining the crowd density for a given time window, that may be used in the present invention.

The step 400, represented in FIGURE 4, may be the step 208 in the method 200 of FIGURE 2.

The step 400 comprises a step 402 determining a total number of unique ID(s) received by the user device of the POI, during the given time window for which the crowd diversity is calculated.

A step 404 calculates a density value, noted V, by dividing the total number of unique ID(s) by a reception range value associated with the user device of the POI. The reception range value is generally associated to the user device model and is provided by the manufacturer of the user device, or deducted from the specification of the user device.

The density value calculated at step 404 is used, in a step 406, to classify the crowd density in a class among several predefined classes for which a crowd density value is defined beforehand.

In a non-limitative example, the following crowd classes may be defined, with for each crowd class the associated crowd density:

| Crowd density value | Crowd density class | Calculated density value V |
|---|---|---|
| 1 | Nearly Empty | $V < 0,05$ person/m$^2$ |
| 2 | Very low | $0,05 < V < 0,2$ person/m$^2$ |
| 4 | low | $0,2 < V < 0,3$ person/m$^2$ |
| 6 | moderate | $0,3 < V < 0,4$ person/m$^2$ |
| 8 | high | $0,4 < V < 1,10$ person/m$^2$ |
| 12 | Very high | $1,0 < V < 2,0$ person/m$^2$ |
| 20 | Extremely high | $0,2 < V$ person/m$^2$ |

Then, the crowd density value is read for the crowd density class. For example, if the calculated density value V is equal to 0,5 person/m$^2$, the crowd density class is "high". Thus, the crowd density value is equal to 8.

Of course, these values and classes are given only as examples and are in no way limitative.

Moreover, step 400, described here with reference to FIGURE 4, is only a non-limitative advantageous example for calculating the crowd density value. Of course, other techniques for calculating crowd density value may be used in the present invention.

FIGURE 5 is a schematic representation of a non-limitative example of a system according to the invention for contamination likelihood assessment for a person of interest (POI).

The system 500, represented in FIGURE 5, comprises a user device 502 intended to be carried out by the Person Of Interest (POI) 504. The user device 502 is configured to receive and process the signals emitted by other user devices, in order to determine and store for each received signal:

- an RSSI value for said received signal, and
- a personal ID contained in, or represented by, said received signal.

To do this, the user device may comprise an antenna 506 for receiving the signals emitted by other user devices, and a software application 508, called client application, installed in the user device 502 and executed by the user device 502 of the POI 504.

In the example shown in FIGURE 5, the client application 508 is configured to carry out all the steps of the risk assessment method according to the invention, such as the steps of the method 200 of the FIGURE 2.

FIGURE 6 is a schematic representation of another non-limitative example of a system according to the invention for contamination likelihood assessment for a person of interest (POI).

The system 600, represented in FIGURE 6, comprises all the components of the system 500 of FIGURE 5.

The system 600 further comprises a central server 602 executing a software application 604, called server application 604, installed in said server 602.

The server 602 is in communication with the user device 502 through a communication network 606, such as the Internet.

In the example shown in FIGURE 6, the client application 508 and the server application 604 are configured to carry out all the steps of the risk assessment method according to the invention, such as the steps of the method 200 of the FIGURE 2.

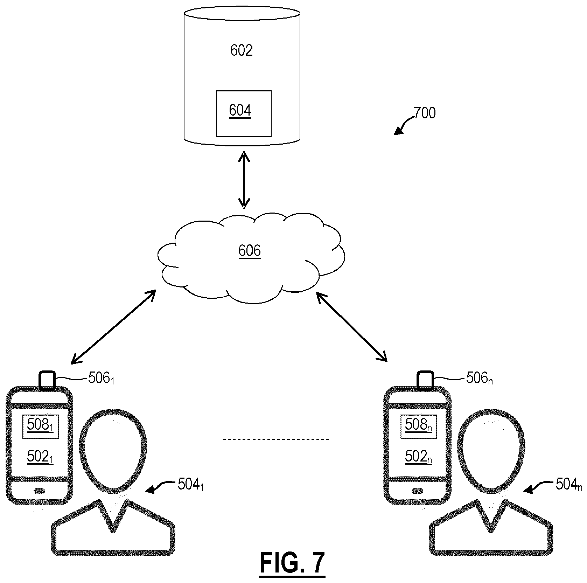

FIGURE 7 is a schematic representation of a non-limitative example of a system according to the invention for detecting persons suspected of contamination, in case of a contamination between persons, in particular in the event of a pandemic.

The system 700 of the FIGURE 7 comprises the central server 602 executing the server application 604, installed in said server 602.

The central server 602 is in communication with several persons $504_1$-$504_n$, each carrying a user device $502_1$-$502_n$ through the communication network 606. Each user device $502_i$ comprises an antenna $506_i$ and a client application $508_i$, like the user device 502 of FIGURES 5 and 6.

Each user device $502_i$ is configured to:

- emit a signal with a personal ID, individual and associated to, the person $504_i$ carrying the user device $502_i$, and
- detect and process signals with personal ID emitted by other user devices, alone or in cooperation with the central server 602, and especially the server application 604.

The system 700 is configured to carry out the likelihood assessment method according to the invention, such as the method 200 of the FIGURE 2, individually for each of the persons $502_1$-$504_n$, who have encountered a contaminated, and/or sick, person. Thus, the system 700 may detect persons suspected of contamination, in case of a contamination between persons, in particular in the event of a pandemic, for a group of several persons.

Of course, the invention is not limited to the examples detailed above. Variants and alternative embodiments may be imagined without departing from the scope of the invention as defined in the claims

**Claims**

1. A method (200) for the evaluation of a contamination likelihood for a person of interest (POI), carrying a user device (502) configured to detect signals with personal IDs emitted by other user devices, said method (200) comprising the following steps:

   - determining (202) a duration, called exposure duration, comprising at least one time window(s) (108), of a predetermined length, during which the user device (502) of said POI detected at least one signal, called contaminated person's signal, emitted from the user device of at least one contaminated person, and
   - for each time window (108), assessing (214) a contamination likelihood for said POI according to:

      ▪ a received signal strength indication (RSSI) value, for the contaminated person's signal, relative to the distance between said contaminated person and the POI, and

- an RSSI threshold value, representing a contamination distance below which contamination is possible;

**characterized in that** said method (200) comprises using at least one crowd attribute characterizing the crowd around said POI during at least one of said time window(s), to adjust the received RSSI value and/or the RSSI threshold value.

2. The method (200) according to the preceding claim, wherein the at least one crowd attribute is used to adjust the RSSI threshold value, said method (200) comprising a step (212) for calculating an adjusted RSSI threshold value as function of:

- said at least one attribute of the crowd, and
- a given RSSI cutoff value, representing a contamination distance for the user device of the POI.

3. The method (200) according to any one of the preceding claim, wherein a first crowd attribute, called crowd diversity, used to adjust the received RSSI value, and/or the RSSI threshold value, relates to the change of persons in the crowd around said POI, during at least one time window.

4. The method (200) according to preceding claim, wherein said method (200) comprises a step (204;300) for determining the crowd diversity for at least one time window (108) and comprising the following operations:

- determining (302) a first list of unique ID(s) received by the user device (502) of the POI, during said time window (108),
- determining (304) a second list of unique ID(s) received by the user device (502) of the POI, during the time window immediately preceding said time window (108), and
- calculating (306) said crowd diversity for said time window (108) as, or as a function of, the symmetric difference between said first and second lists of unique ID(s).

5. The method (200) according to any one of claims 3 or 4, wherein, for at least one, and in particular for each, time window, an average value, called mean crowd diversity, of the crowd diversities determined for several time windows, and in particular for all time windows, of the exposure duration, is used to adjust the received RSSI value, and/or the RSSI threshold value.

6. The method (200) according to anyone of the preceding claims, wherein a second crowd attribute, called crowd density, used to adjust the received RSSI value, and/or the RSSI threshold value, relates to the density of persons in the crowd around said POI during at least one time window (108).

7. The method (200) according to the preceding claim, wherein said method comprises a step (208;400) for determining the crowd density for at least one time window (108) and comprising the following operations:

- Determining (402) a total number of unique ID(s) received by the user device (502) of the POI, during said time window (108,
- Calculating (404) a density value by dividing said total number of unique ID(s) by said range value, representing the reception range of the POI's user device (502), and
- determining (406,408) a crowd density value for said time window (108), as a function of said density value.

8. The method (200) according to the preceding claim, wherein, for at least one, and in particular for each, time window (108), an average value, called mean crowd density, of the crowd densities for several, and in particular for all, time windows (108) of the exposure duration, is used to adjust the received RSSI value, and/or the RSSI threshold value.

9. The method (200) according to the claims 2, 5 and 8, wherein the adjusted is determined by the following formula:

$$\text{Adjusted RSSI threshold} = \text{base RSSI Cutoff} + \frac{base\ RSSI\ Cutoff}{mean\ Crowd\ Diversity^2} * mean\ crowd\ density$$

with:

- base RSSI Cutoff : the RSSI cutoff value for a contamination distance for the POI's user device,
- RSSI threshold : the adjusted RSSI threshold value,
- *mean Crowd Diversity* : the average crowd diversity value, and
- *mean crowd density* : the average crowd density value.

**10.** A computer program comprising instructions which, when executed by at least one data processing device, cause said at least one data processing device to carry out the method (200) according to any one of the preceding claims.

**11.** A method for detecting persons suspected of contamination, in case of a contamination between persons, in particular in the event of a pandemic, said method comprising:

- equipping persons ($504_1$-$504_n$) with user devices ($502_1$-$502_n$), each configured to emit a signal with a personal ID and detect signals with personal ID emitted by other user devices, and
- evaluating the contamination likelihood of at least one of said persons ($504_1$-$504_n$) by the method (200) according to the method according to any one of the claims 1 to 9.

**12.** A system (500;600) for the evaluation of a contamination likelihood for a person of interest (POI), said system (500;600) comprising:

- a user device (502), provided for being carried out by the POI (504) and configured to detect signals with personal IDs emitted by other user devices, and
- a computer program (508), called client application, installed in said user device (502);

configured to carry out the steps of the method (200) according to any one of the claims 1 to 9.

**13.** The system (600) according to the preceding claim, wherein said system (600) also comprises:

- a central server (602), in communication with the user device (502), and
- a computer program (604), called server application, installed in said central server (602);

said server application being configured to carry out, together with the user device (502) and the client application (508) installed therein, the steps of the method (200) according to any one of the claims 1 to 9.

**14.** A system (700) for detecting persons suspected of contamination, in case of a contamination between persons, in particular in the event of a pandemic, said system (700) comprising:

- for each of said persons ($504_1$-$504_n$):

  ▪ a user device ($502_1$-$502_n$) configured to emit a signal with a personal ID and detect signals with personal ID emitted by other user devices ($502_1$-$502_n$), and
  ▪ a computer program ($508_1$-$508_n$), called client application, installed in said user device ($502_1$-$502_n$);

- and for several, in particular for all persons ($504_1$-$504_n$):

  ▪ a central server (602), in communication with the user device ($502_1$-$502_n$), and
  ▪ a computer program (604), called server application, installed in said central server (602);

configured to carry out the steps of the method (200) according to any one of the claims 1 to 9, for at least one of said persons.

**Patentansprüche**

**1.** Verfahren (200) für die Beurteilung einer Kontaminationswahrscheinlichkeit für eine Person von Interesse (POI), die eine Benutzervorrichtung (502) trägt, die konfiguriert ist, um Signale mit persönlichen IDen zu erfassen, die durch andere Benutzervorrichtungen emittiert werden, das Verfahren (200) umfassend die folgenden Schritte:

- Bestimmen (202) einer Dauer, die als Belichtungsdauer bezeichnet wird, umfassend mindestens ein Zeitfenster (108), von einer zuvor bestimmten Länge, während der die Benutzervorrichtung (502) der POI mindestens ein Signal erfasst, das als das Signal einer kontaminierten Personals bezeichnet wird, das von der Benutzervorrichtung mindestens einer kontaminierten Person emittiert wird, und

- für jedes Zeitfenster (108), Einschätzen (214) einer Kontaminationswahrscheinlichkeit für die POI gemäß:

■ einem Empfangssignalstärkeindikator-Wert (RSSI-Wert) für das Signal der kontaminierten Person relativ zu dem Abstand zwischen der kontaminierten Person und der POI, und
■ einem RSSI-Schwellenwert, der einen Kontaminationsabstand darstellt, unter dem Kontamination möglich ist;

**dadurch gekennzeichnet, dass** das Verfahren (200) ein Verwenden von mindestens einem Menschenmengenattribut umfasst, das die Menschenmenge um die POI während mindestens eines von dem/den Zeitfenster(n) kennzeichnet, um den empfangenen RSSI-Wert und/oder RSSI-Schwellenwert einzustellen.

2. Verfahren (200) nach dem vorstehenden Anspruch, wobei das mindestens eine Menschenmengenattribut verwendet wird, um den RSSI-Schwellenwert einzustellen, das Verfahren (200) umfassend einen Schritt (212) zum Berechnen eines eingestellten RSSI-Schwellenwerts als Funktion von:

- dem mindestens einem Attribut der Menschenmenge und
- einem gegebenen RSSI-Cutoff-Wert, der einen Kontaminationsabstand für die Benutzervorrichtung der POI darstellt.

3. Verfahren (200) nach einem der vorstehenden Ansprüche, wobei ein erstes Menschenmengenattribut, das als Menschenmengendiversität bezeichnet wird, das verwendet wird, um den empfangenen RSSI-Wert und/oder RSSI-Schwellenwert einzustellen, sich während mindestens eines Zeitfensters auf die Änderung von Personen in der Menschenmenge um die POI bezieht.

4. Verfahren (200) nach dem vorstehenden Anspruch, wobei das Verfahren (200) einen Schritt (204; 300) zum Bestimmen der Menschenmengendiversität für mindestens ein Zeitfenster (108) umfasst und umfassend die folgenden Vorgänge:

- Bestimmen (302) einer ersten Liste von eindeutigen ID(en), die durch die Benutzervorrichtung (502) der POI während des Zeitfensters (108) empfangen werden,
- Bestimmen (304) einer zweiten Liste von eindeutigen ID(en), die durch die Benutzervorrichtung (502) der POI während des Zeitfensters empfangen werden, das dem Zeitfenster (108) unmittelbar vorausgeht, und
- Berechnen (306) der Menschenmengendiversität für das Zeitfenster (108) als oder in Abhängigkeit von dem symmetrischen Unterschied zwischen der ersten und der zweiten Liste von eindeutigen ID(en).

5. Verfahren (200) nach einem der Ansprüche 3 oder 4, wobei, für mindestens ein, und insbesondere für jedes, Zeitfenster, ein Durchschnittswert, der als mittlere Menschenmengendiversität bezeichnet wird, der Menschenmengendiversitäten, der für mehrere Zeitfenster und insbesondere für alle Zeitfenster der Belichtungsdauer bestimmt wird, verwendet wird, um den empfangenen RSSI-Wert und/oder RSSI-Schwellenwert einzustellen.

6. Verfahren (200) nach einem der vorstehenden Ansprüche, wobei ein zweites Menschenmengenattribut, das als Menschenmengendichte bezeichnet wird, das verwendet wird, um den empfangenen RSSI-Wert und/oder RSSI-Schwellenwert einzustellen, sich auf die Dichte von Personen in der Menschenmenge um die POI während mindestens eines Zeitfensters (108) bezieht.

7. Verfahren (200) nach dem vorstehenden Anspruch, wobei das Verfahren einen Schritt (208; 400) zum Bestimmen der Menschenmengendichte für mindestens ein Zeitfenster (108) umfasst und umfassend die folgenden Vorgänge:

- Bestimmen (402) einer Gesamtzahl von eindeutigen ID(en), die durch die Benutzervorrichtung (502) der POI während des Zeitfensters (108 empfangen werden,
- Berechnen (404) eines Dichtewerts durch Dividieren der Gesamtzahl von eindeutigen ID(en) durch den Bereichswert, der den Empfangsbereich der Benutzervorrichtung (502) der POI darstellt, und
- Bestimmen (406, 408) eines Menschenmengendichtewerts für das Zeitfenster (108) in Abhängigkeit von dem Dichtewert.

8. Verfahren (200) nach dem vorstehenden Anspruch, wobei, für mindestens ein, und insbesondere für jedes, Zeitfenster (108), ein Durchschnittswert, der als mittlere Menschenmengendichte bezeichnet wird, der Menschenmengendichten für mehrere, und insbesondere für alle, Zeitfenster (108) der Belichtungsdauer verwendet wird, um den empfangenen RSSI-Wert und/oder RSSI-Schwellenwert einzustellen.

9. Verfahren (200) nach den Ansprüchen 2, 5 und 8, wobei das Eingestellte durch die folgende Formel bestimmt wird:

$$\text{Eingestellter RSSI} - \text{Schwellenwert} = \text{Basis} - \text{RSSI} - \text{Cutoff} +$$

$$\frac{Basis - RSSI - Cutoff}{mittlere\ Menschenmengendiversität^2} * mittlere\ Menschenmengendichte$$

mit:

- Basis-RSSI-Cutoff: der RSSI-Cutoff-Wert für einen Kontaminationsabstand für die Benutzervorrichtung der POI,
- RSSI-Schwellenwert: der eingestellte RSSI-Schwellenwert,
- *mittlere Menschenmengendiversität*: der durchschnittliche Menschenmengendiversitätswert, und
- *mittlere Menschenmengendichte*: der durchschnittliche Menschenmengendichtewert.

10. Computerprogramm, umfassend Anweisungen, die, wenn sie durch mindestens eine Datenverarbeitungsvorrichtung ausgeführt werden, die mindestens eine Datenverarbeitungsvorrichtung veranlassen, das Verfahren (200) nach einem der vorstehenden Ansprüche auszuführen.

11. Verfahren zum Erfassen von Personen, bei denen Kontamination vermutet wird, im Falle einer Kontamination zwischen Personen, insbesondere im Falle einer Pandemie, das Verfahren umfassend:

- Ausstatten von Personen ($504_1$-$504_n$) mit Benutzervorrichtungen ($502_1$-$502_n$), die jeweils konfiguriert sind, um ein Signal mit einer persönlichen ID zu emittieren und Signale mit persönlicher ID zu erfassen, die durch andere Benutzervorrichtungen emittiert werden, und
- Beurteilen der Kontaminationswahrscheinlichkeit mindestens einer der Personen ($504_1$-$504_n$) durch das Verfahren (200) gemäß dem Verfahren nach einem der Ansprüche 1 bis 9.

12. System (500; 600) für die Beurteilung einer Kontaminationswahrscheinlichkeit für eine Person von Interesse (POI), das System (500; 600) umfassend:

- eine Benutzervorrichtung (502), die bereitgestellt ist, um durch die POI (504) ausgeführt zu werden, und konfiguriert ist, um Signale mit persönlichen IDen zu erfassen, die durch andere Benutzervorrichtungen emittiert werden, und
- ein Computerprogramm (508), das als Client-Anwendung bezeichnet wird, das in der Benutzervorrichtung (502) installiert ist;
das konfiguriert ist, um die Schritte des Verfahrens (200) nach einem der Ansprüche 1 bis 9 auszuführen.

13. System (600) nach dem vorstehenden Anspruch, wobei das System (600) auch umfasst:

- einen zentralen Server (602) in Kommunikation mit der Benutzervorrichtung (502), und
- ein Computerprogramm (604), das als Server-Anwendung bezeichnet wird, das in dem zentralen Server (602) installiert ist;
wobei die Server-Anwendung konfiguriert ist, um zusammen mit der Benutzervorrichtung (502) und der darin installierten Client-Anwendung (508) die Schritte des Verfahrens (200) nach einem der Ansprüche 1 bis 9 auszuführen.

14. System (700) zum Erfassen von Personen, bei denen Kontamination vermutet wird, im Falle einer Kontamination zwischen Personen, insbesondere im Falle einer Pandemie, das System (700) umfassend:

- für jede der Personen ($504_1$-$504_n$):

  ■ eine Benutzervorrichtung ($502_1$-$502_n$), die konfiguriert ist, um ein Signal mit einer persönlichen ID zu emittieren und Signale mit persönlicher ID zu erfassen, die durch andere Benutzervorrichtungen ($502_1$-

$502_n$) emittiert werden, und

- ■ ein Computerprogramm ($508_1$-$508_n$), das als Client-Anwendung bezeichnet wird, das in der Benutzer-vorrichtung ($502_1$-$502_n$) installiert ist;

- und für mehrere, insbesondere für alle Personen ($504_1$-$504_n$):

- ■ einen zentralen Server (602) in Kommunikation mit der Benutzervorrichtung ($502_1$-$502_n$), und
- ■ ein Computerprogramm (604), das als Server-Anwendung bezeichnet wird, das in dem zentralen Server (602) installiert ist;

das konfiguriert ist, um die Schritte des Verfahrens (200) nach einem der Ansprüche 1 bis 9 für mindestens eine der Personen auszuführen.

## Revendications

1. Procédé (200) d'évaluation d'une probabilité de contamination pour une personne d'intérêt (POI), portant un dispositif utilisateur (502) configuré pour détecter des signaux avec des identifiants personnels émis par d'autres dispositifs utilisateur, ledit procédé (200) comprenant les étapes suivantes :

   - une détermination (202) d'une durée, dite durée d'exposition, comprenant au moins une ou des fenêtre(s) temporelle(s) (108), d'une longueur prédéterminée, au cours de laquelle le dispositif utilisateur (502) de ladite POI a détecté au moins un signal, dit signal de personne contaminée, émis depuis le dispositif utilisateur d'au moins une personne contaminée, et
   - pour chaque fenêtre temporelle (108), une évaluation (214) d'une probabilité de contamination pour ladite POI selon :

      • une valeur d'indication de force de signal reçu, RSSI, pour le signal de personne contaminée, par rapport à la distance entre ladite personne contaminée et la POI, et
      • une valeur seuil de RSSI, représentant une distance de contamination en dessous de laquelle la contamination est possible ;

   **caractérisé en ce que** ledit procédé (200) comprend l'utilisation d'au moins un attribut de foule caractérisant la foule autour de ladite POI pendant au moins l'une parmi ladite ou lesdites fenêtre(s) temporelle(s), pour ajuster la valeur de RSSI reçue et/ou la valeur seuil de RSSI.

2. Procédé (200) selon la revendication précédente, dans lequel l'au moins un attribut de foule est utilisé pour ajuster la valeur seuil de RSSI, ledit procédé (200) comprenant une étape (212) de calcul d'une valeur seuil de RSSI ajustée en fonction :

   - dudit au moins un attribut de la foule, et
   - d'une valeur de coupure de RSSI donnée, représentant une distance de contamination pour le dispositif utilisateur de la POI.

3. Procédé (200) selon l'une quelconque des revendications précédentes, dans lequel un premier attribut de foule, dit diversité de foule, utilisé pour ajuster la valeur de RSSI reçue, et/ou la valeur seuil de RSSI, concerne le changement de personnes dans la foule autour de ladite POI, pendant au moins une fenêtre temporelle.

4. Procédé (200) selon la revendication précédente, dans lequel ledit procédé (200) comprend une étape (204 ; 300) de détermination de la diversité de foule pour au moins une fenêtre temporelle (108) et comprenant les opérations suivantes :

   - une détermination (302) d'une première liste d'identifiant(s) unique(s) reçu(s) par le dispositif utilisateur (502) de la POI, pendant ladite fenêtre temporelle (108),
   - une détermination (304) d'une deuxième liste d'identifiant(s) unique(s) reçu(s) par le dispositif utilisateur (502) de la POI, pendant la fenêtre temporelle précédant immédiatement ladite fenêtre temporelle (108), et
   - un calcul (306) de ladite diversité de foule pour ladite fenêtre temporelle (108) comme, ou en fonction de, la différence symétrique entre lesdites première et deuxième listes d'identifiant(s) unique(s).

**5.** Procédé (200) selon l'une quelconque des revendications 3 ou 4, dans lequel, pour au moins une, et en particulier, pour chaque fenêtre temporelle, une valeur moyenne, dite diversité de foule moyenne, des diversités de foule déterminées pour plusieurs fenêtres temporelles, et en particulier pour toutes les fenêtres temporelles, de la durée d'exposition, est utilisée pour ajuster la valeur de RSSI reçue, et/ou la valeur seuil de RSSI.

**6.** Procédé (200) selon l'une quelconque des revendications précédentes, dans lequel un deuxième attribut de foule, dit densité de foule, utilisé pour ajuster la valeur RSSI reçue, et/ou la valeur seuil RSSI, concerne la densité de personnes dans la foule autour de ladite POI pendant au moins une fenêtre temporelle (108).

**7.** Procédé (200) selon la revendication précédente, dans lequel ledit procédé comprend une étape (208 ; 400) de détermination de la densité de foule pour au moins une fenêtre temporelle (108) et comprenant les opérations suivantes :

- une détermination (402) d'un nombre total d'identifiant(s) unique(s) reçu(s) par le dispositif utilisateur (502) de la POI, pendant ladite fenêtre temporelle (108),
- un calcul (404) d'une valeur de densité en divisant ledit nombre total d'identifiant(s) unique(s) par ladite valeur de plage, représentant la plage de réception du dispositif utilisateur de la POI (502), et
- une détermination (406, 408) d'une valeur de densité de foule pour ladite fenêtre temporelle (108), en fonction de ladite valeur de densité.

**8.** Procédé (200) selon la revendication précédente, dans lequel, pour au moins une, et en particulier pour chaque fenêtre temporelle (108), une valeur moyenne, dite densité de foule moyenne, des densités de foule pour plusieurs, et en particulier pour toutes les fenêtres temporelles (108) de la durée d'exposition, est utilisée pour ajuster la valeur de RSSI reçue, et/ou la valeur seuil de RSSI.

**9.** Procédé (200) selon les revendications 2, 5 et 8, dans lequel la valeur seuil de RSSI ajustée est déterminée par la formule suivante :

$$\text{Seuil de RSSI ajusté} = \text{coupure de RSSI de base} + \frac{\text{coupure de RSSI de base}}{\text{diversité de foule moyenne}^2} * \text{densité de foule moyenne}$$

avec :

- coupure de RSSI de base : la valeur de coupure de RSSI pour une distance de contamination pour le dispositif utilisateur de la POI,
- seuil de RSSI : la valeur seuil de RSSI ajustée,
- diversité de foule moyenne : la valeur de diversité de foule moyenne, et
- densité de foule moyenne : la valeur de densité de foule moyenne.

**10.** Programme d'ordinateur comprenant des instructions qui, lorsqu'elles sont exécutées par au moins un dispositif de traitement de données, amènent ledit au moins un dispositif de traitement de données à mettre en oeuvre le procédé (200) selon l'une quelconque des revendications précédentes.

**11.** Procédé de détection de personnes soupçonnées de contamination, en cas de contamination entre des personnes, en particulier en cas de pandémie, ledit procédé comprenant :

- un équipement de personnes ($504_1$-$504_n$) avec des dispositifs utilisateur ($502_1$-$502_n$), chacun configuré pour émettre un signal avec un identifiant personnel et détecter des signaux avec un identifiant personnel émis par d'autres dispositifs utilisateur, et
- une évaluation de la probabilité de contamination d'au moins une desdites personnes ($504_1$-$504_n$) par le procédé (200) selon l'une quelconque des revendications 1 à 9.

**12.** Système (500 ; 600) pour l'évaluation d'une probabilité de contamination pour une personne d'intérêt (POI), ledit système (500 ; 600) comprenant :

- un dispositif utilisateur (502), fourni pour être porté par la POI (504) et configuré pour détecter des signaux avec des identifiants personnels émis par d'autres dispositifs utilisateur, et

- un programme informatique (508), dit application client, installé dans ledit dispositif utilisateur (502) ;

configuré pour mettre en oeuvre les étapes du procédé (200) selon l'une quelconque des revendications 1 à 9.

**13.** Système (600) selon la revendication précédente, dans lequel ledit système (600) comprend également :

- un serveur central (602), en communication avec le dispositif utilisateur (502), et
- un programme informatique (604), dit application serveur, installé dans ledit serveur central (602) ;

ladite application serveur étant configurée pour mettre en oeuvre, conjointement avec le dispositif utilisateur (502) et l'application client (508) installée dessus, les étapes du procédé (200) selon l'une quelconque des revendications 1 à 9.

**14.** Système (700) de détection de personnes soupçonnées de contamination, en cas de contamination entre des personnes, en particulier en cas de pandémie, ledit système (700) comprenant :

- pour chacune desdites personnes ($504_1$-$504_n$) :

• un dispositif utilisateur ($502_1$-$502_n$) configuré pour émettre un signal avec un identifiant personnel et détecter des signaux avec un identifiant personnel émis par d'autres dispositifs utilisateur ($502_1$-$502_n$), et
• un programme informatique ($508_1$-$508_n$), dite application client, installée dans ledit dispositif utilisateur ($502_1$-$502_n$) ;

- et pour plusieurs, notamment pour toutes les personnes ($504_1$-$504_n$) :

• un serveur central (602), en communication avec le dispositif utilisateur ($502_1$-$502_n$), et
• un programme informatique (604), dit application serveur, installé dans ledit serveur central (602) ;

configuré pour mettre en oeuvre les étapes du procédé (200) selon l'une quelconque des revendications 1 à 9, pour au moins une desdites personnes.

**FIG. 1**

Determine exposure duration comprising at least one time window ⌐202

Calculate crowd diversity for each time window of the exposure duration ⌐204

Calculate mean crowd diversity ⌐206

Caculate crowd density for each time window of the exposure duration ⌐208

Calculate mean crowd density ⌐210

Calculate adjusted RSSI threshold value ⌐212

Assess contamination likelihood for each time window ⌐214

200

## FIG. 2

Determining a first list of unique ID for the time window ⌐302

Determining a second list of unique IDs for the previous time window ⌐304

Calculate symmetric difference between the two list ⌐306

300

## FIG. 3

## FIG. 4

```
Determining first list of unique ID for the time    ──402
window
          │
          ▼
Calculating density value V                         ──404
          │
          ▼
Identifying crowd density class                     ──406
          │
          ▼
Read crowd density value associated to crowd        ──408
class
```

400

## FIG. 5

506

502

508

500

504

## FIG. 6

600

602

604

606

506

508

502

504

**FIG. 7**